# EUROPEAN PATENT SPECIFICATION

(11) **EP 2 090 326 B1**
(45) Date of publication and mention of the grant of the patent: **18.09.2013**
(21) Application number: 09152685.5
(22) Date of filing: 12.02.2009
(51) Int. Cl.: A61M 5/32

(54) **Safety pen needle with cannula protector element**
Sicherheits-Injektionsstiftnadel mit Schutzelement für die Nadel
Aiguille-stylo d'injection de sécurité avec élément protecteur de l'aiguille

(30) Priority: 13.02.2008 IT MI20080218
(43) Date of publication of application: 19.08.2009
(73) Proprietor: ARTSANA S.p.A., 22070 Grandate (Como) (IT)
(72) Inventor: De Zolt, Dario, 22070, Appiano Gentile (Como) (IT)
(74) Representative: Ripamonti, Enrico

(56) References cited:
- EP-A- 1 177 803
- US-A1- 2005 096 599

## Description

The present invention relates to a safety pen needle in accordance with the introduction to the main claim.

A pen needle is known to be a device used generally by patients suffering from diabetes for daily insulin injections. This device comprises a needle carrier to be screw-connected to an insulin pen in which an insulin-containing cartridge is placed. The device is used both in a domestic environment (self-administration, i.e. the patient self-administering an appropriate drug dose) and in a hospital environment (a medical operator injects the patient).

A pen needle traditionally comprises mainly:
- a metal cannula provided with two points; one at each end: the purpose of the first, known as the "patient point", being to cut the epidermis, to enable the cannula to be inserted into the patient's skin; the purpose of the second, internal to the needle carrier and known as the "vial point" being to perforate the insulin cartridge membrane once the device is mounted on a pen. In this manner, during injection, the insulin can pass from the cartridge to the patient through the cannula;
- the aforesaid needle carrier, of plastic material, mainly cylindrical in shape and having a form such as to be able to be connected (by a threaded ring or a thread present on the inner surface of said needle carrier) to the threaded end of an insulin pen;
- two container elements, namely a main element of greater dimensions, the function of which is to completely cover the pen device or needle before use, and a secondary element of smaller dimensions, disposed in a position such as to cover the cannula "patient point";
- a seal (of paper or other material) positioned on the open end of the primary container, to ensure device sterility.

During the use of a traditional pen needle certain problems have appeared, including the fact that:
- before injection the patient, on seeing the cannula, could become agitated or be afraid of encountering excessive pain during the injection;
- the device is intended for once-only use, i.e. after use the pen needle must be immediately disposed of in accordance with existing regulations. However, research has shown that a patient may reuse the device, with the risk of infection or greater pain and trauma, to the damage of the patient;
- while manipulating the device, but also during its disposal, it can happen that the medical operator assisting the patient, or a refuse disposal operative, or a family member, may become pricked by the needle, with possible contraction of serious and dangerous infections.

To obviate the aforesaid problems, pen needles are known provided with internal movable elements arranged to securely cover the cannula after its use, to hence prevent undesired contact with the patient or other person. Pen needles are also known in which the cannula, prior to use, is covered by a protector element so as not to be visible to the patient, said protector element being however movable axially to the cannula to enable normal use of the pen needle by the patient.

By way of example, US6986760 and US2005/0277897 describe a pen needle and a protection system for cannula safety. The pen needle is of the aforesaid type and comprises a protector element in which a helical spring is present acting at one end on the element itself and at the other end on a coupling member rigid with the needle carrier. The spring always acts on the protector element and counteracts the movement which it makes when the patient injects the medicament by pressing precisely this element (from which a free end of the cannula can emerge) against his/her body, causing the cannula to penetrate into this latter to release the medicament.

The presence of the spring renders the injection operation difficult as the spring opposes the movement of the protector element which enables the cannula to emerge. This fact can lead to mistaken injection or to greater pain for the patient, as the injection is made more difficult.

In addition the known needle device or pen is of complicated construction precisely because the spring always acts on the protector element.

Another known solution is described in WO03/045480 which describes a pen needle of the aforesaid type containing a helical spring positioned within the device to cooperate indirectly with a needle protector element or directly with a locking member inside the element. This latter is arranged to move simultaneously with the element during injection (to free the free end of the cannula which is to cooperate with the patient's body) and to return with the element onto the cannula by the thrust of the spring, so as to lock said protector element in a position such as to cover the free end of the cannula.

This known solution comprises a number of movable parts always subjected, directly or indirectly, to the action of the spring; this renders product assembly relatively complicated. Moreover, as in the case of the aforestated prior US patents, even with this last solution the patient has to overcome the force of the spring to make the injection, this rendering the operation uncomfortable.

US 2005/0096599 refers to a safety device for a syringe having features equivalent to those in the preamble of the main claim. EP 1177803 describes an hypodermic syringe having an internal protector element for its needle after the use.

An object of the present invention is to provide a pen needle of the aforesaid type which solves the aforestated problems regarding the use of the needle by representing an improvement over the already known solutions.

A particular object of the invention is to provide a pen needle enabling the cannula to be hidden from the patient's view, such as to reduce the sense of anxiety and fear, hence rendering the injection apparently less traumatic and painful for the patient.

Another object is to make it impossible to reuse the device, hence eliminating all those problems deriving from this practice.

A further object is to eliminate the risk of the patient operator coming into contact with the needle after use, hence ensuring safety of those operators who nurse the patient or of anybody who may come into contact with the device after its use.

Another object is to provide a safety pen needle of the stated type which is of simple construction and use, such that the needle insertion into the patient happens delicately, and moreover is of small overall size, its use not negatively affecting the functionality of the pen needle itself.

These and other objects, which will be apparent to the expert of the art, are attained by a pen needle in accordance with the accompanying claims.

The present invention will be better understood from the accompanying drawings, which are provided by way of non-limiting example and in which:
Figure 1 is a perspective view of a pen needle which is not part of the invention;
Figure 2 is a perspective view of the pen needle of Figure 1 ready for use (the insulin pen is not shown) with some parts omitted for greater clarity;
Figure 3 is a perspective exploded view of the pen needle of Figure 1;
Figure 4 is a section on the line 4-4 of Figure 2;
Figure 5 is an exploded perspective view of a detail of the pen needle of Figure 1;
Figure 6 is a section on the line 6-6 of Figure 5;
Figure 7 is an exploded perspective view of parts of the pen needle of Figure 1;
Figure 8 is an exploded perspective view of different parts of the pen needle of Figure 1;
Figure 9 is a view similar to Figure 4, but showing a stage in the use of the pen needle of Figure 1;
Figure 10 is a perspective view of the pen needle during the stage of use of Figure 9;
Figure 11 is a perspective view of the pen needle on termination of its use;
Figure 12 is an exploded perspective view of another pen needle which is not part of the invention;
Figure 13 is an exploded perspective view of a different part of the pen needle of Figure 12;
Figure 14 is a section through the pen needle of Figure 12 shown assembled;
Figure 15 is a side view of a further pen needle which is not part of the invention;
Figure 16 is a perspective view of a variant of the pen needle of Figure 15;
Figure 17 is a section on the line 17-17 of Figure 15;
Figure 18 is a perspective view of a variant of the pen needle which is not part of the invention;
Figure 19 is a cross-section through this latter Figure taken on the plane of Figure 17;

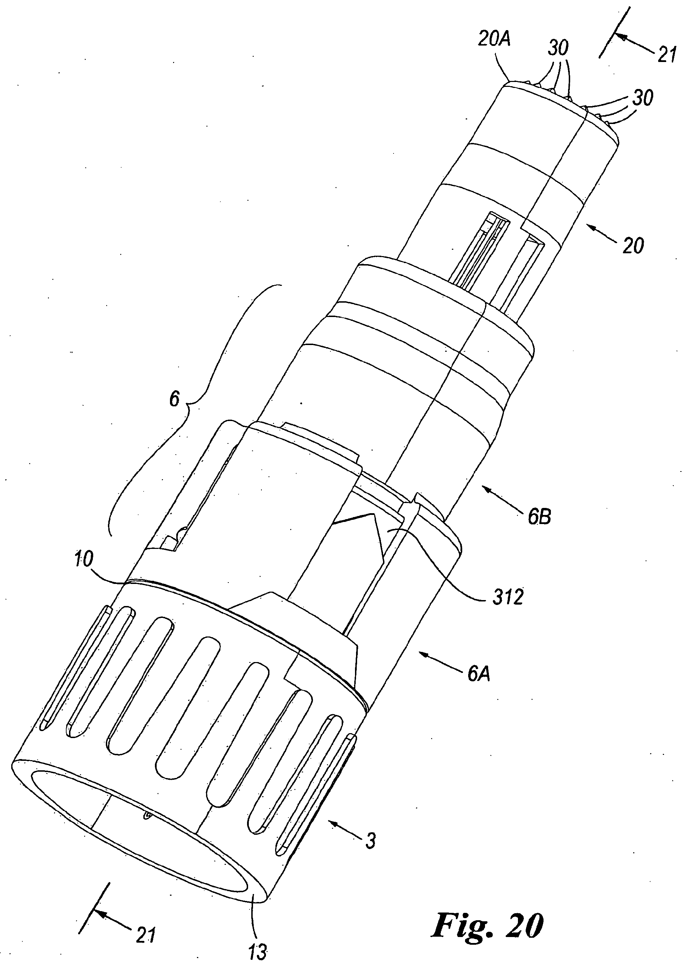

Figure 20 is a perspective view of a pen needle according to the invention;
Figure 21 is a section on the line 21-21 of Figure 20;

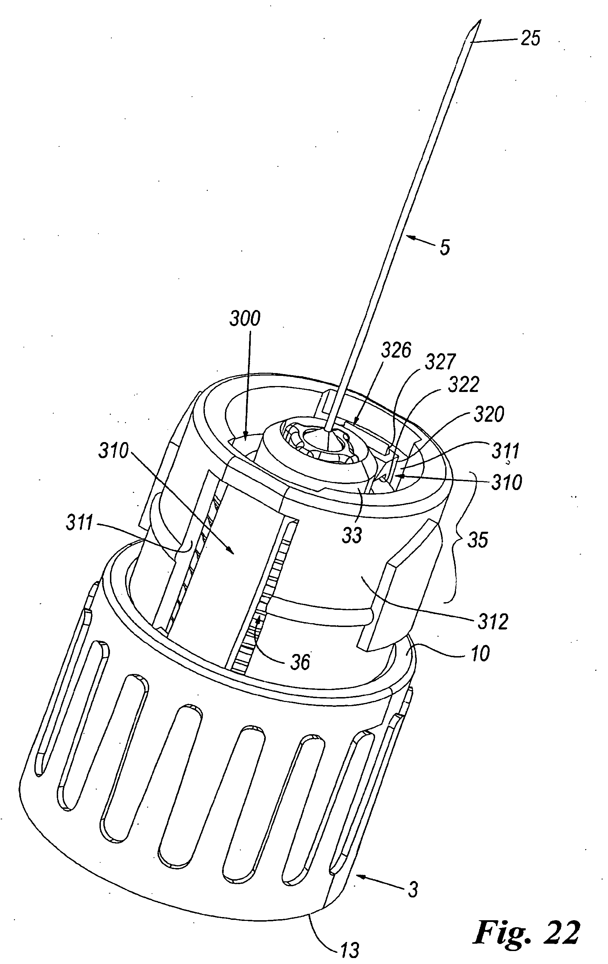

Figure 22 is a perspective view of a portion of the pen needle of Figure 2;

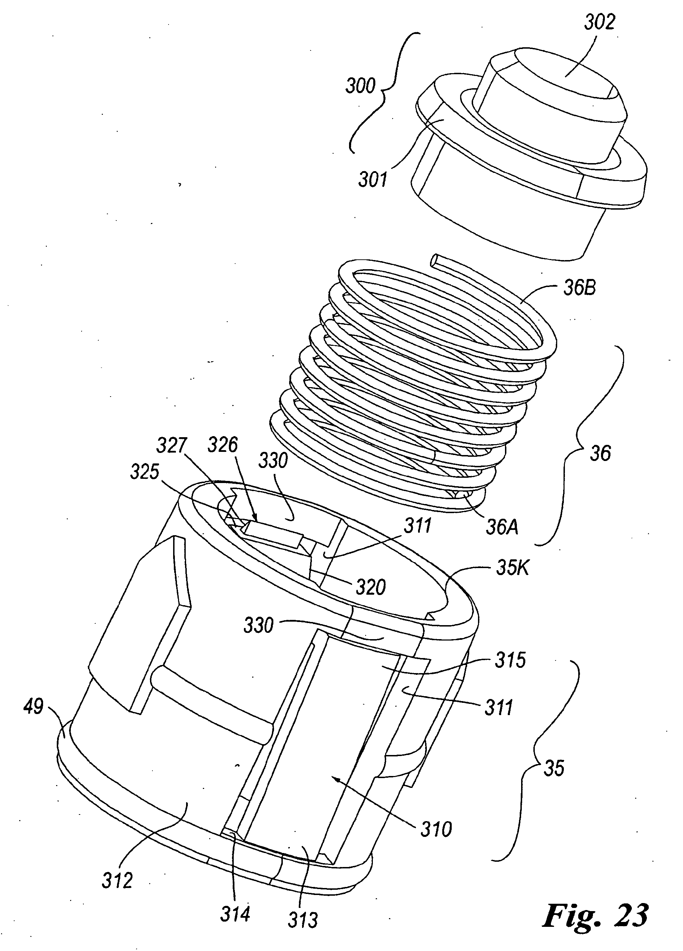

Figure 23 is an exploded view of the portion of Figure 22, with a part omitted for greater clarity;

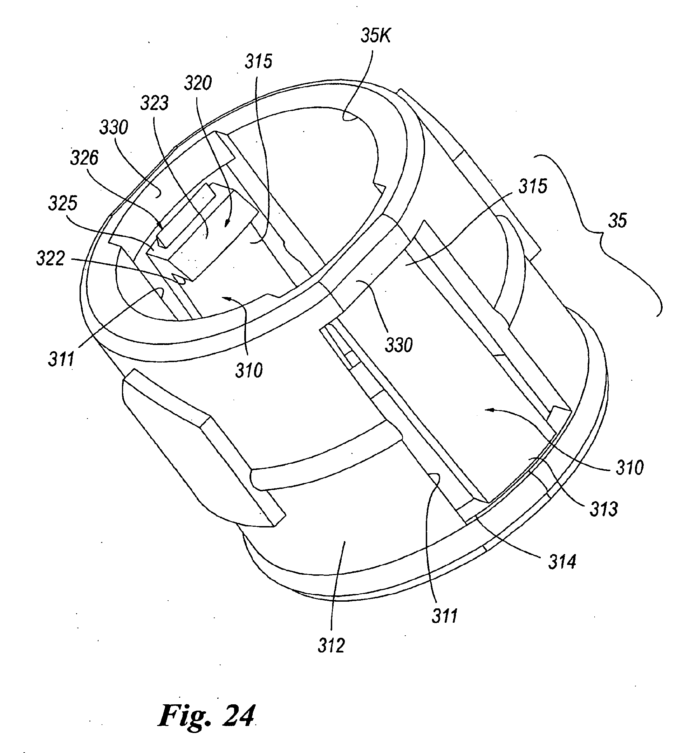

Figure 24 is a perspective view of a part of the portion of Figure 22; and
Figure 25 is a view similar to Figure 21 but with the pen needle in a different stage of use.
With reference to said Figures from 1 to 11, a pen needle is indicated overall by 1 and comprises a body 1A to be connected, by screwing, to a pen in which a container (not shown) is inserted for medicaments, for example (but not necessarily) for diabetics. This body 1A is covered for manipulation purposes I(for example for its connection to the pen) by a primary container element 2 which is superposed on a needle carrier 3 with which a cannula 5 is associated in any known manner. A cover element 6 presenting two portions 6A, 6B of different cross-section is also coupled to the needle carrier 3. The needle carrier 3 presents a cup-shaped first end 10 with a raised edge 11. An annular recess 9 is present on the inside of said edge. The needle carrier 3 comprises a second end 13, opposite to the first, and having an edge 14 which is at least partially threaded internally (at 15). This thread 15 enables the needle carrier 3 to be connected to a threaded end of the pen (not shown), to enable it to be fixed to this latter.

Within the hollow cover element 6, and in particular within its portion 6B (tapered towards the free end 6H open at 66 and defined by elastic arms 67), a cylindrical protection element 20 is movable comprising a first end 20A external to said element 6 and a second end 20B internal to this latter. This second end 20B is flanged at 21 to cooperate with an internally bent edge 22 (bent towards the longitudinal axis W of the pen needle 1) of the end 6H of the cover element 6, to prevent complete withdrawal of the protector element 20 through the aperture 66 of the cover element 6.

The first end 20A of the element 20 is open at 24 to allow a first end 25 of the cannula (defining its "patient point") to pass to the outside of the pen needle 1. The cannula 5 also presents an opposing end 26 (or "vial point") on the inside of the needle carrier 3 and arranged to cooperate with a medicament container (not shown) placed in the pen.

On the outer edge of the first end 20A, the protector element 20 comprises a plurality of slightly pointed protuberances 30. These protuberances 30 make contact with the patient's skin to "mask" the sensation of pain by the injection and make this latter less painful.

The cannula 5 is partially inserted into a tubular body 33 of the needle carrier 3 positioned along the axis W of the pen. A cylindrical container 35, in which a helical spring 36 is present, is positioned about said body 33, within a recess 34 of the first end 10 of the needle carrier 3 defined by the edge 11, and within the portion 6a of the cover element 6.

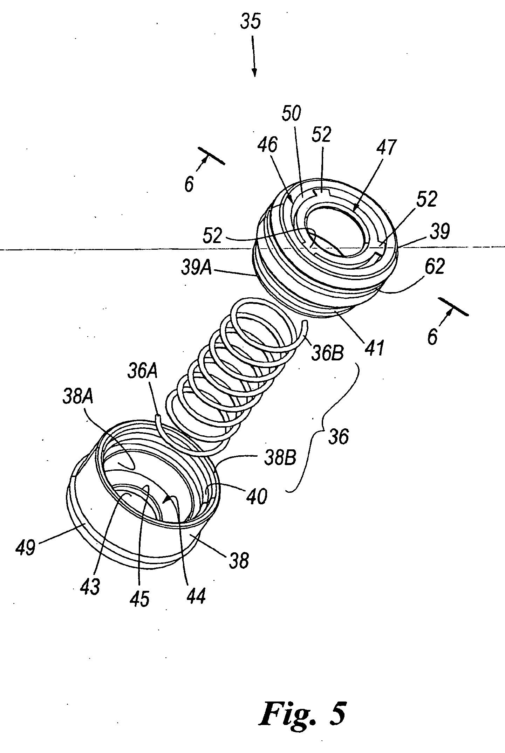

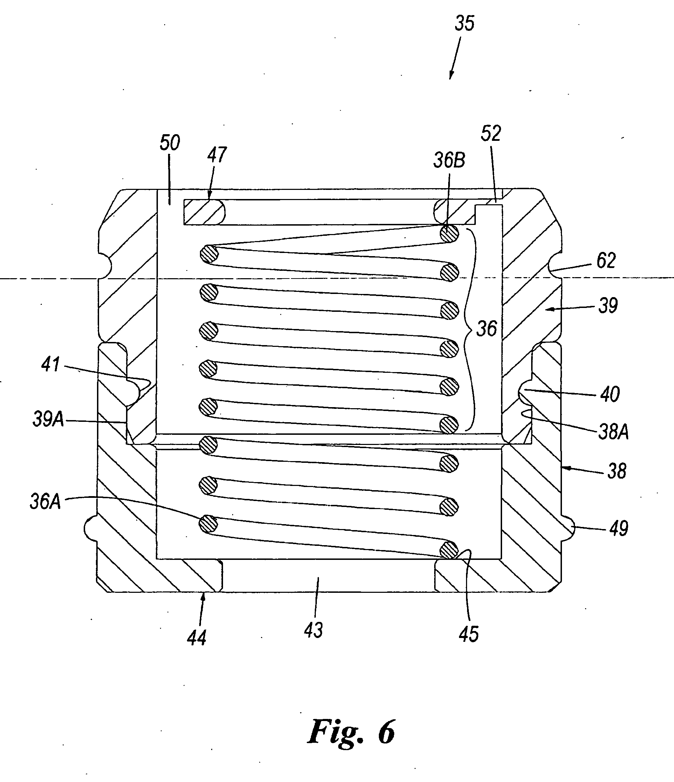

More specifically, the container 35 comprises a first hollow cylindrical portion or support 38 and a cover or second hollow cylindrical portion 39 snap-fitted to the first portion by coupling an inner collar 40 of the first portion (present on its wall 38A) to an annular seat 41 of the second portion formed inside a wall 39A thereof. This coupling enables a container 35 to be obtained which is independent of the rest of the components of the pen needle 1, and can also be preassembled with the corresponding spring 36. Obviously, the container 35 can be also assembled in any other known manner (for example by interference). With particular reference to Figures 5 and 6, these show how the support 38, of cup shape and open at that end 38B facing the portion 39, has an aperture 43 within a base 44 to rest on the needle carrier 3. This aperture is defined by an annular portion 45 on which a first end 36A of the spring 36 rests. The second end 36B of this spring rests on a ring 47 positioned at a first end 46 of the cup-shaped second portion 39 of the container 35, which is open at its second end (that facing the second portion 38). This ring is present at an aperture 50 of said first end 46 and is coupled to the wall 39A of the portion 39 by breakable arms or bridges 52 of variable number and shape (for example, three in number as in the figures), but of small thickness.

By virtue of this construction, as stated, the container 35 can be preassembled with the internal spring 36 and mounted on the tubular body 33 of the needle carrier 3, this tubular body 33 being inserted into the aperture 43 and into the ring 47. The container 35 is coupled to the needle carrier 3 in the recess 34 by coupling thereto its collar 49, which projects from the portion 38 and cooperates with the recess 9 in the edge 11 of the end 10 of the needle carrier 3. These elements could also be coupled together in other ways known in the state of the art (for example by interference).

The pen needle above cited therefore comprises various elements: the primary container 2, internally hollow at 2A, in which the needle carrier 3 is inserted, preassembled with the cannula 5 and container 35, and the cover element 6; into this latter, before assembling the needle carrier 3, the protector element 20 is inserted, the protuberances 30 of which, positioned on its first end 20A, rest against the closed end 2C of the container 2. In this manner, the protector element 20 becomes positioned correctly with respect to the cover element 6. In this respect, as the protector element 20 rests against the end 2C of the container 2, the assembly of the various parts 20, 6, 35, 3 of the pen needle bringing this protector element 20 into a position which is external to or projecting onto the cover element 6.

It should be noted that the container 35 also snap-fits onto the cover element 6. In this respect, the portion 39 of said container 35 presents an external annular recess 62 into which there projects a collar 63 provided on the inside of the portion 6A of the element 6. This connection between the container 35 and the element 6 can also be achieved in any other known manner, for example by interference.

A removable closure paper 60 (or a seal of other material) closes the open or free end 2B of the container.

The pen needle is used in the following manner.

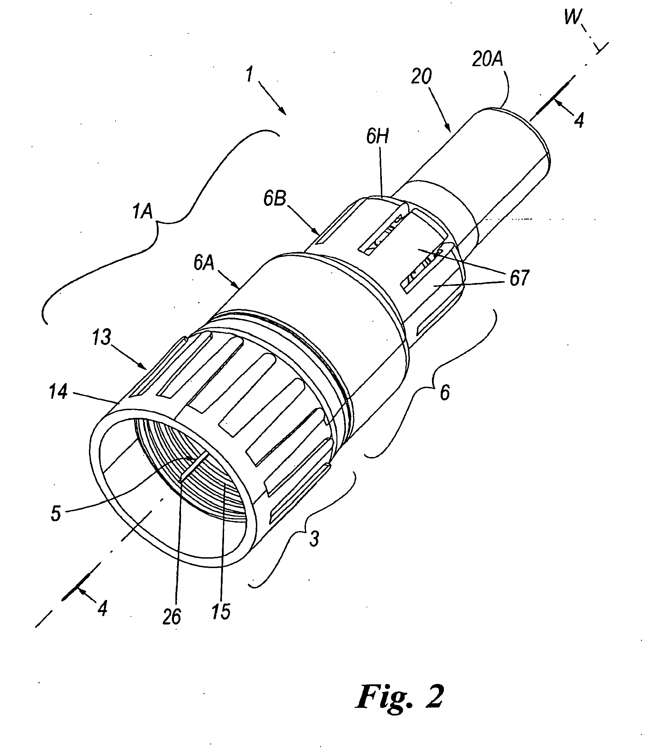

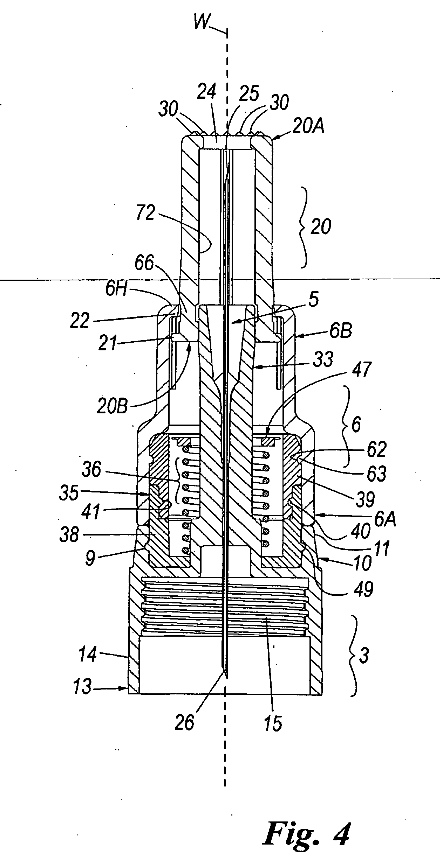

After removing the closure paper 60, an operator fits the device or pen needle 1 onto a normal insulin pen. At this point, the primary container 2 is removed and the device appears as in Figures 2 and 4. While resting the end 20A of the movable protector element 20 (projecting considerably from the cover element 6) on the skin, the operator exerts a light pressure to make the device approach the skin and hence initiate the injection (on him/herself in the case of self-administration, or on a patient).

At this point, the movable protector element 20 begins to slide within the cover element 6, to expose the cannula 5 which can hence penetrate into the skin.

Before use, the movable protector element 20 remains in a fixed position because of its interference with the tapered portion 6H of the external cover element 6 and because of the cooperation between a number of ribs 72, present on the inside of the element 20, and the tubular body 33. In this manner, the movable protector element is unable to move unless sufficient pressure is applied thereto, and the cannula 5 remains hidden within the protector element. However, as the ribs 72 of the protector element 20 interfere only with a first portion of the body 33 of the needle carrier 3, the pressure necessary to move the protector element 20 is minimal. In fact, the aforesaid interference is such as to maintain the protector element 20 in position prior to use, but not such as to hinder injection. Said protector element 20 can hence slide easily within the element 6, thus enabling the injection to be easily carried out.

During injection, the lower end of the movable protector element 20 reaches the ring 47 of the cover element 6. Because of the small thickness of the arms 52 which connect it to the wall 39A of the portion 39, a light pressure applied to it (caused by the movement of the element 20) causes the arms to break.

Consequently, the movement of the protector element 20, having made contact with the ring 47, urged by the action of the operator on the pen, results in breakage of the arms 52.

To facilitate breakage of said arms, a weakening (not shown) of suitable shape can be provided on them.

At this point, the ring 47 is no longer fixed to the cover element 6 and can be moved, urged by the movable protector element 20 against the spring 36. This spring hence compresses to enable the protector element 20 to proceed on its travel and enables the cannula 5 to penetrate completely into the patient's skin.

When the movable protector element 20 is completely inside the cover element 6, the cannula 5 has completely penetrated into the skin and hence the drug can be completely injected. in this position, the spring 36 is completely compressed.

When the injection is finished, the cannula can be extracted from the patient's body. During this extraction, the spring 36 extends to urge the movable protector element 20 to the outside of the cover element 6 through its end aperture. As the constraint imposed by the ring 47 no longer exists, and which previously held it partially compressed, the spring 36 is free to extend completely and to urge the movable protector element beyond the cover element.

The spring 36 pushes the protector element as far as a point in which an undercut 80 present in its outer surface 81 exceeds the free (tapered) end 6H of the cover element; in this manner the undercut abuts against that end, and the movable protector element is again unable to slide within the cover element. In the meantime, the flange 21 cooperating with this end 6H prevents the protector element from "withdrawing" from the element 6. Hence the cannula 5 cannot be exposed and the risk of accidental pricking and the possibility of reuse are nullified.

To facilitate activation of this safety mechanism, the free end of the cover element has elastic arms 67 to give the end 6H greater elasticity.

The spring 36 must evidently be of sufficient length to be able to snap-operate this safety mechanism irreversibly.

Advantageously, a display element (for example a coloured band) can be provided in proximity to the undercut of the protector element; its display indicates that the protector element has reached the safety protection position for the cannula 5. Apertures and windows 90 can also be provided close to the undercut to act as this display element.

In a variant of the above described pen needle, the inner surface of the movable protector element 20 does not present any ribs, whereas some ribs are present along the tubular body 33 of the needle carrier 3. The function of these ribs is identical to that of the ribs present on the aforedescribed movable protector element.

Figures from 12 to 14 show a variant of the invention. In these figures, in which parts corresponding to those of the already described figures are indicated by the same reference numerals, the elongate body 33 comprises a collar 100 in proximity to its free end, said collar having an undercut 101 to cooperate with a corresponding abutment 102 present in proximity to the second end 20B, within the element 6, of the movable protector element 20 to block its exit from the container element 6 (urged by the spring 36).

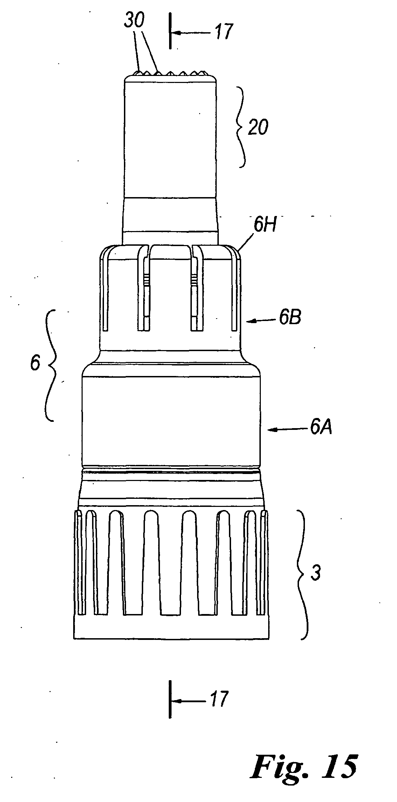

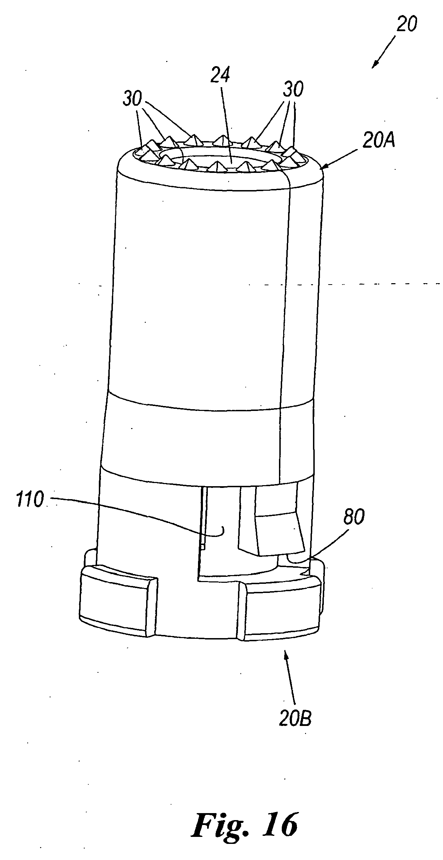

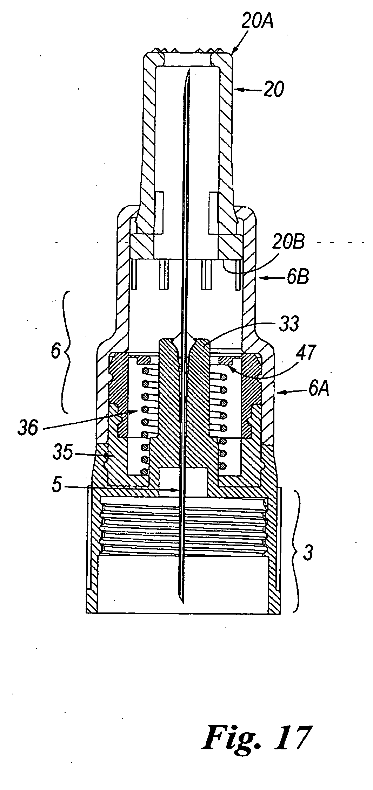

A further embodiment of a pen needle, which is not part of the invention is shown in Figures 15, 16 and 17, in which parts corresponding to those of the already described figures are indicated by the same reference numerals.

In this embodiment, the elongate body 33 of the needle carrier 3 is of lesser length than the corresponding body of the other already described embodiments.

This variant also has a different shape of undercut 80 of the movable protector element 20. In this variant, the element is provided with a cavity 110 in which this undercut can move, and is flexible to achieve even simpler activation for the previously described irreversible safety mechanism. This element 20 interferes via its second end 20B with the external cover element 6 when in the initial rest position as this internal part has larger dimensions than the portion 6B of said element 6. This enables said initial position to be maintained when the needle 1 is not used.

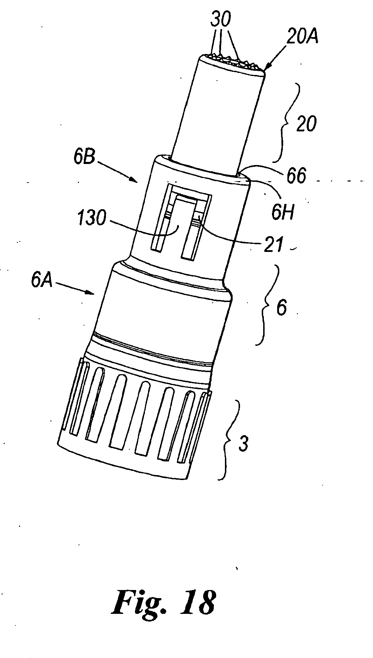

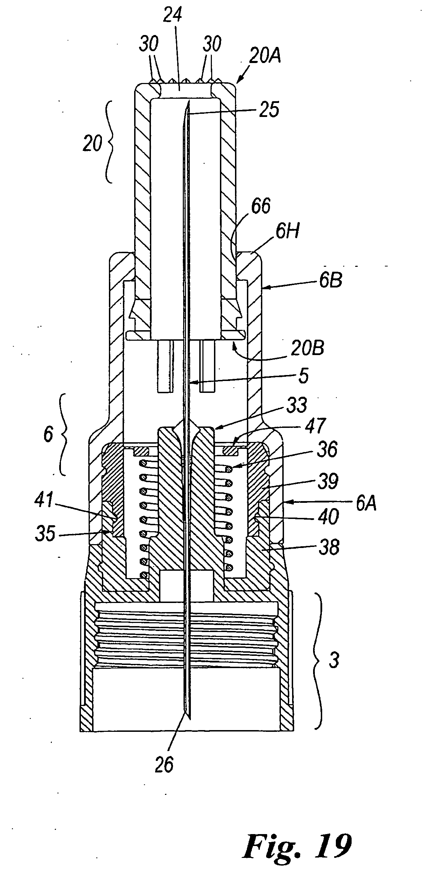

Another variant is shown in Figures 18 and 19, in which parts corresponding to those of the already described figures are indicated by the same reference numerals.

In this embodiment, the elongate body 33 of the needle carrier 3 has the same length as that described in the aforedescribed Figures 15-17.

This variant also has a different shape for each undercut 80 present on the protector element 20. These undercuts are shaped as a cavity totally similar, in shape and function, as the already described cavity 110. With each cavity there interfere elastic arms 130 close to the internally bent edge 22 of the free end 6H of the cover element 6. This enables the safety position to be maintained after using the needle 1.

Figures from 20 to 25, in which parts corresponding to those of the already described figures are indicated by the same reference numerals, show a pen needle according to the invention. In this variant, the cylindrical container 35 is in one piece containing the spring 36 and a body 300 of substantially cylindrical shape. The body 300 has a collar 301 and an axial through bore 302 so that it can be disposed about the tubular body 33 of the needle carrier 3.

The spring 36 is positioned between the collar 301 and the interior of the recess 34 at the end 10 needle carrier 3; this spring acts on said collar to press the body 300 in the direction of the protector element 20, i.e. towards that open end 35K of the container 35 which faces this latter.

In the condition prior to the use of the pen needle, the body 300 is inside the container 35 and is maintained therein by elastic arms 310 present in longitudinal slots (i.e. disposed along the axis W) 311 provided in the lateral part 312 of the container 35 defining the inner cavity of this latter. These arms 310 are secured at a first end 313 to a part 314 of the container 35 and have their second end 315 elastically movable relative to said wall 312 within the slots 311.

Each arm 310 has its second end 315 shaped with an undercut. In other words, this end presents a portion 320 bent preferably (or substantially preferably) at an angle to the arm 310, to define with this latter the undercut 322, said portion having a free inclined surface part 323 facing towards the interior of the container 35. The end 315 terminates with a flat part 325 from which a tab 326 projects above the arm 315 in its longitudinal direction, and has a right-angled triangular cross-section (or other equivalent form) with its inclined side 327 facing outwards from the container.

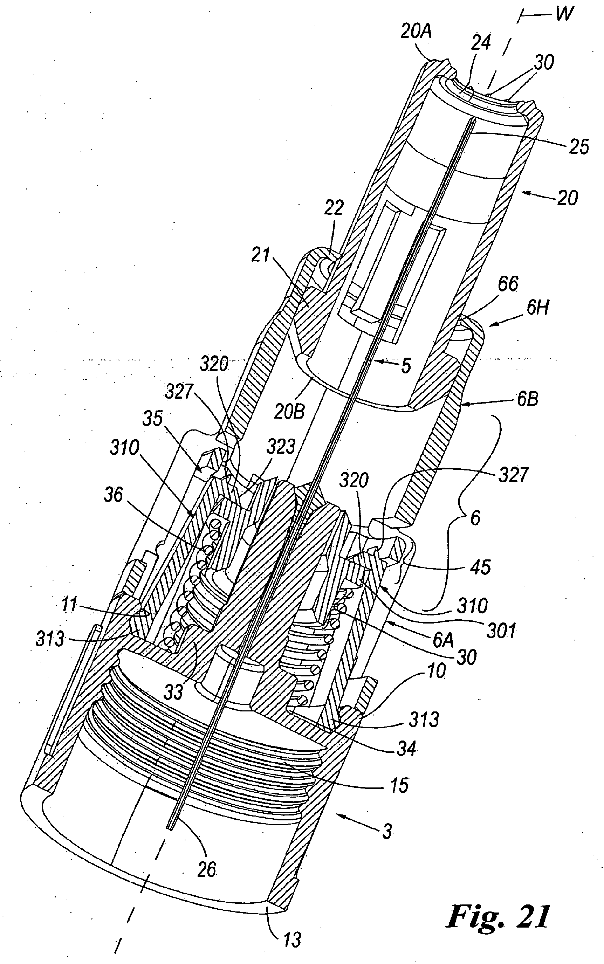

The tab 326 is arranged to cooperate with a bridge 330 which closes the corresponding slot 311 above the arm 310. This bridge maintains the arm 310 in its condition bent towards the outside of the container 35 after the protector element 20 has cooperated therewith on re-entering the cover element 6. This cooperation takes place between the end 20B of the element 20, flared inwards towards the axis W of Figure 21, and the portion 320 of each arm when the element 20 re-enters the element 6.

In this respect, on inserting the needle into the patient, the arms 310 cooperate with the collar 301 of the body 300 to retain it inside the container 35. In particular (see Figure 21), the cooperation between said arms 20 and the collar results in compression of the spring 36.

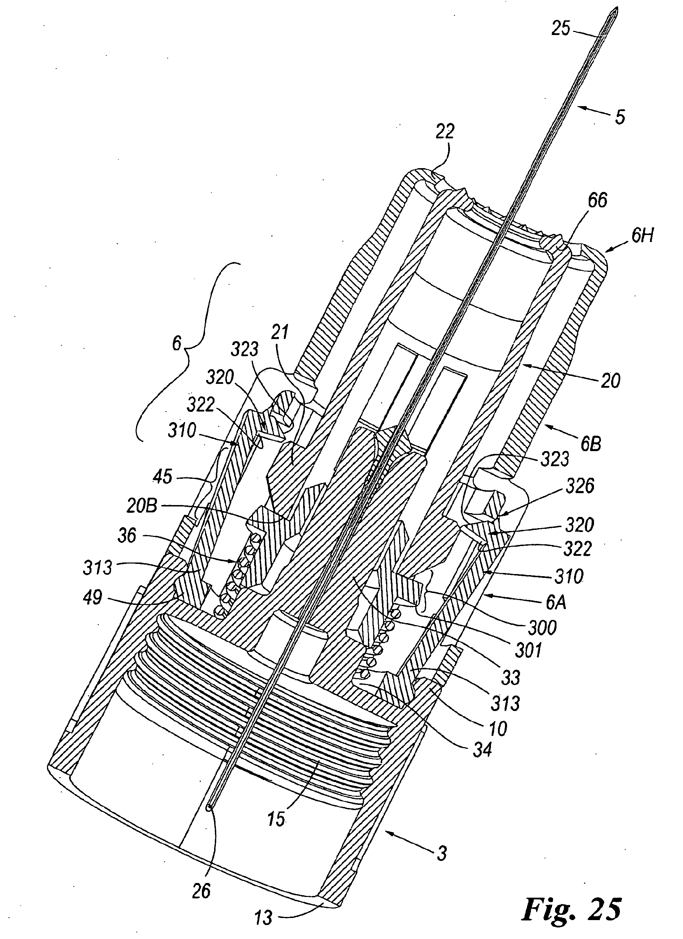

When the needle penetrates the patient's body, the element 20 re-enters the cover element 6 (in the described manner) until its end 20B contacts the inclined surface 323 of each arm 310. At this point, the sliding of said end along said inclined surfaces 323 causes the arms 310 to withdraw from the axis W of the pen needle; this withdrawal causes the tabs 326 of said arms to slide, via their inclined side 327, on the corresponding bridge 330 with consequent deformation of the relative arms, so that each tab 326 overcomes said bridge. It follows that the tab abuts on the relative bridge after overcoming this latter, with consequent locking of the relative arm in a position spaced from the axis W (see Figure 25).

By said movement, the element 20 comes into contact with the body 300, to move it within the cylindrical container 35 towards the needle carrier 3. This results in compression of the spring 36.

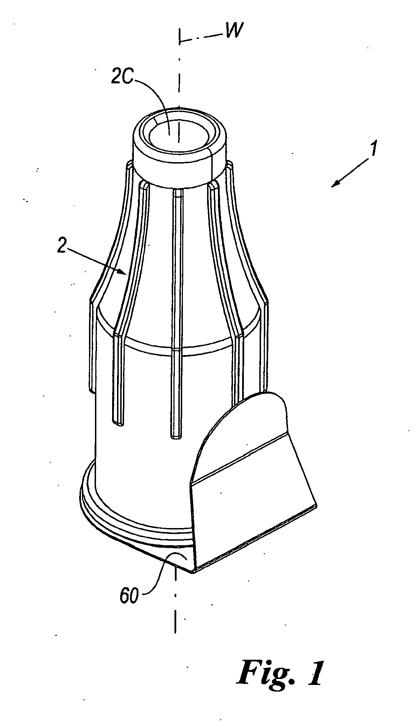

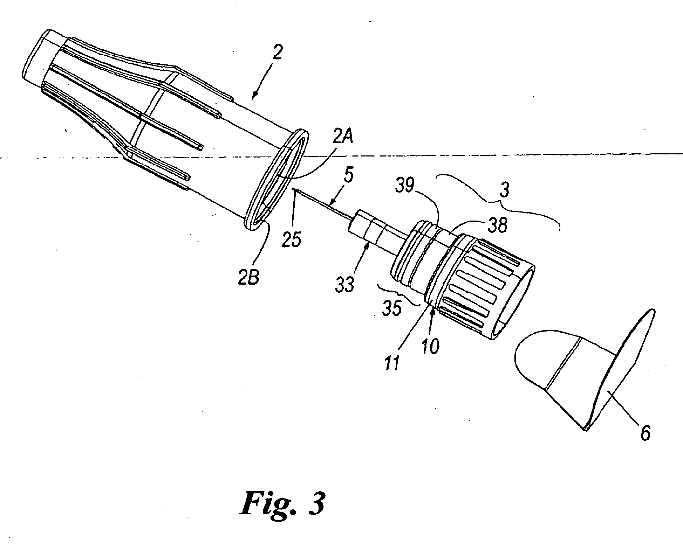

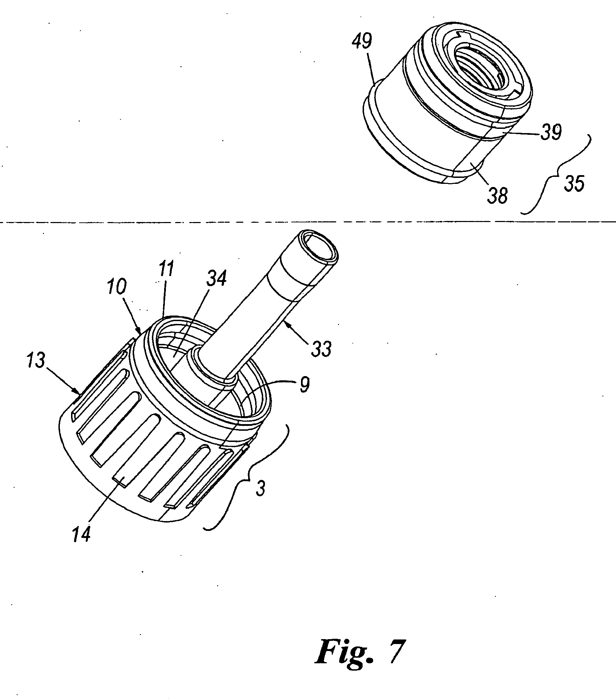

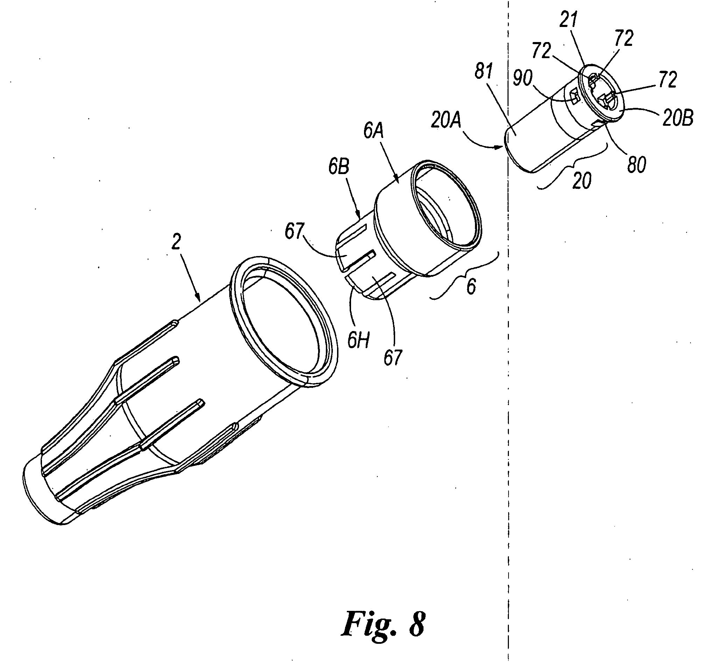

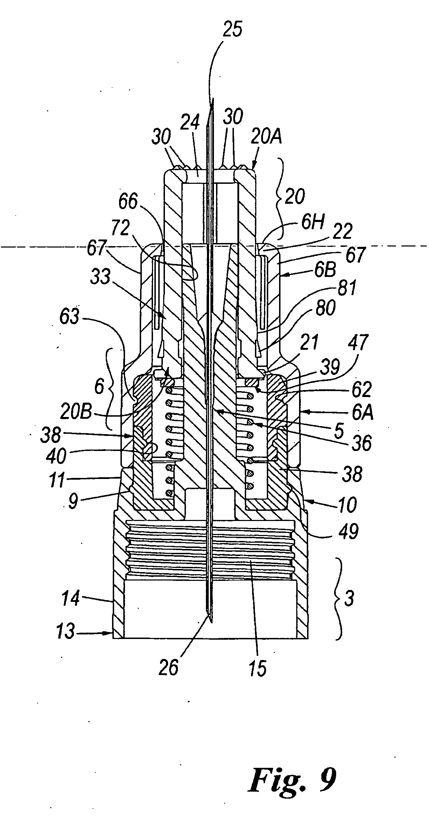

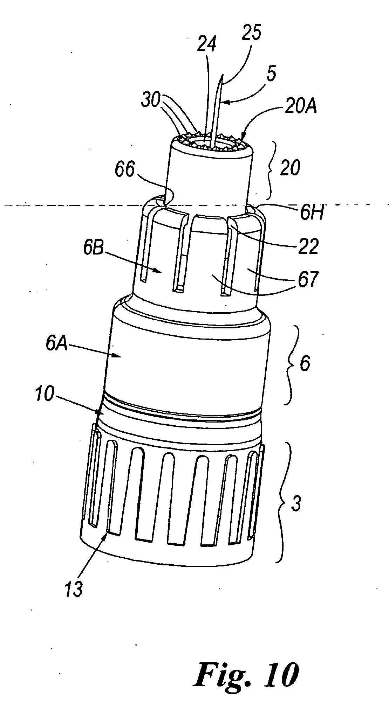

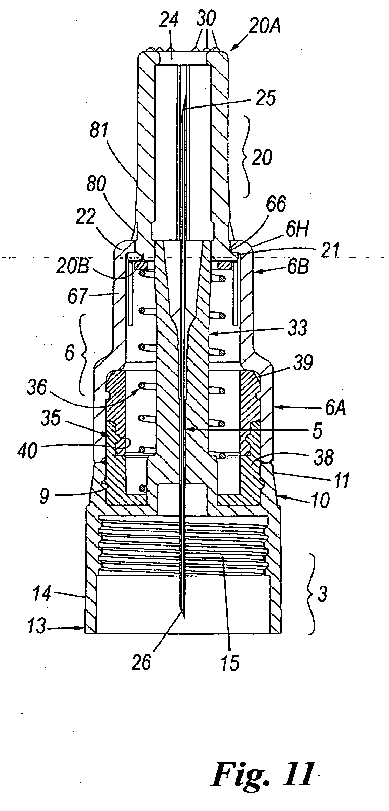

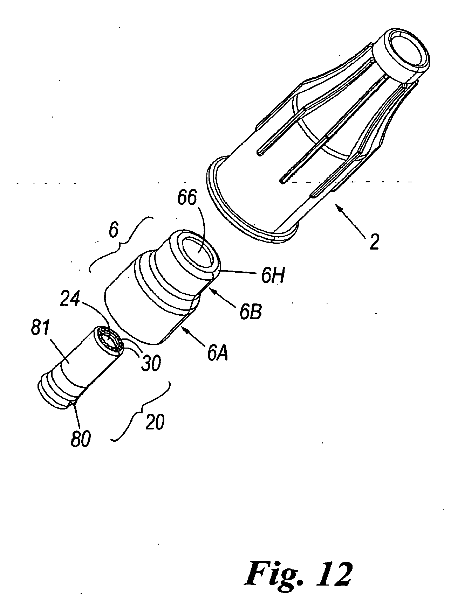

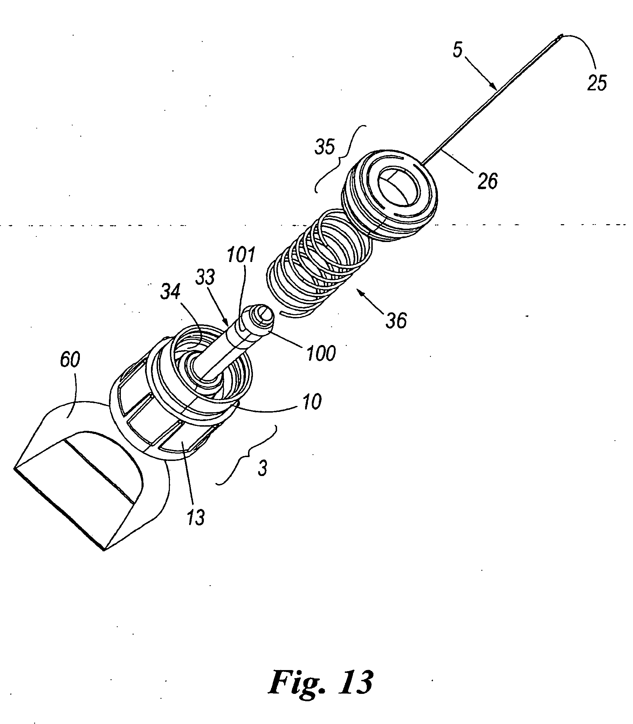

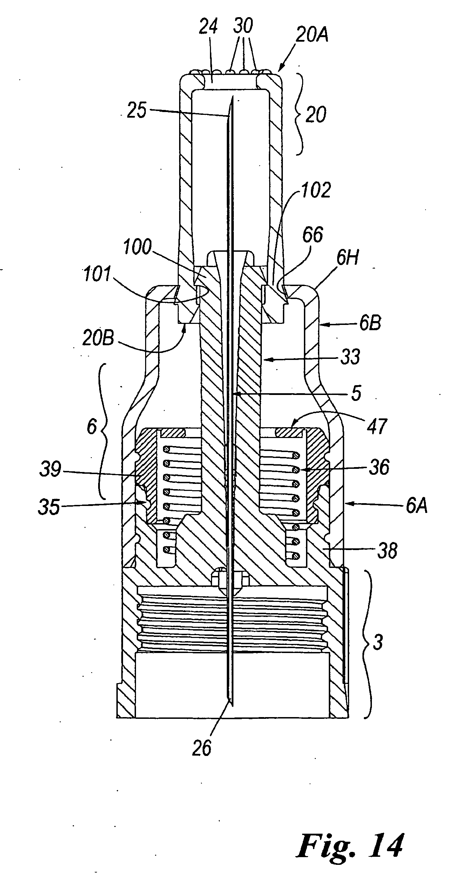

When the injection is complete, the removal of the needle 5 from the patient with withdrawal of the element 6 from the patient's body enables the element 20 to move relative to said element 6, this latter making its withdrawal movement under the thrust of the spring 36. In this respect, this latter acts on the collar 301 of the body 300 and hence by urging this latter out of the container 35 (as it is no longer retained by the arm 310) it causes said element 6 to thrust the element 20 (resting on the body 300) to the outside. This movement results in the element 20 becoming superposed on the needle 5 to protect a medical operator or the user against contact with the first end or "patient point" thereof. The withdrawal movement of the protector element 20 terminates, as in the other embodiments of the pen needle which are not part of the present invention described in figures 1 to 19, by it resting its end 20A on the edge 22 of the element 6.

Various embodiments of the pen needle according to the invention have been described; others are however possible and are to be considered as falling within the scope of the ensuing claims.

## Claims

1. A pen needle to be connected to a container or pen with which a vial of medicament is associated, said needle (1) comprising:
- a metal cannula (5) provided with two points (25, 26), one at each end: the purpose of the first (25), known as the "patient point", being to cut the epidermis, to enable the cannula (5) to be inserted into the patient's skin; the purpose of the second (26), internal to a needle carrier (3) and known as the "vial point", being to perforate the medicinal vial when the pen needle (1) is secured to the pen, this enabling the medicament to be transferred, during the material injection, from the vial to a patient through the cannula (5);
- the aforesaid needle carrier (3), to be connected to said pen;
- a primary container (2), the purpose of which is to receive the entire pen needle (1) prior to use;
- a cover element (6), connected to the needle carrier (3) and positioned about the cannula (5);
- a protector element (20), movable within the cover element (6) and arranged to emerge from this latter so as to cover the first cannula (5) point after its extraction from the patient on termination of the injection, in order to avoid accidental contact with said point (25) by an operator making the injection or by the actual patient, and to prevent re-use of the device;
- an elastic element (36) arranged to press on said protector element (20) such as to enable it to move on termination of the injection and during extraction of the cannula (5) from the patient, said movement causing said element to cover the first point (25) of said cannula (5), locking means (47, 300, 310) being provided for said elastic element (36) such as to prevent its action on said protector element (20), at least until a moment successive to the commencement of introduction of the cannula (5) into the epidermis, said locking means (47; 300, 310) being removable after said moment in which said introduction commenced, **characterised in that** the protector element (20) comprises a first end (20A) external to said cover element (6) and a second end (20B) internal to this latter, the **protector element** (20A) projecting **considerably,** from the cover element (6) and covering the cannula (5) **when the needle pen is ready for use (figure 2, 4), said first end (20A)** on the patient skin **when an operator initiates the injection,** said protector element (20) freely easily sliding within the cover element (6) to expose the cannula (5) **which hence** penetrates into the skin until the protector second end (20B) contacts the locking means, said locking means (300, 310) being removed when the **needle penetrates the patient's body and the protector element (20) enters the cover element (6) by said movement the elastic element (36) resulting compressed so that, when the injection is complete, the removal of the needle (5) form the patient enables the protector element (20) to move relative to said cover element (6) under the trust of the elastic element (36), said protector element so moving** out of the cover element and superimpose the cannula (5).

2. A pen needle as claimed in claim 1, **characterised in that** the locking means comprise an internally hollow container member (35) presenting at least one member (310) elastically movable within a corresponding seat (311) provided in a wall (312) of said container body (35) which defines the inner cavity of this latter, said movable member (310) defining a member for blocking the movement of a body (300) with which a second end (36B) of said elastic element (36) cooperates, said movable blocking member (310) cooperating with the protector element (20) when positioned inside the cover element (6), such as to free the body (300) cooperating with the elastic element and enable this latter to urge said body (300), with consequent action of this latter on said protector element such as to move it to the outside of the cover element (6) and position it about the first point or patient point (25) of the cannula (5).

3. A pen needle as claimed in claim 2, **characterised in that** said body (300) presents a collar (301) with which the second end (36B) of the elastic element (36) cooperates, a first end (36A) of said elastic element (36) being inside the container body (35) and resting on it, said at least one movable member (310) being an arm connected at its first end (313) to said container body (35) and having a second end (315) freely disposed within the corresponding seat (311) provided in the wall (312) of said container body, said second end presenting a portion (320) bent at an angle towards the interior of said container body (35) relative to the corresponding arm (310) and defining with this latter an undercut (322) arranged to rest on the collar (301) of the body (300) cooperating with said elastic element (36), said bent portion (320) presenting an inclined surface (323) tapering towards the interior of the container body (35) and slidingly cooperating with a tapered end (20B) of the protector element (20) internal to the cover element (6), this cooperation occurring as the protector element (20) re-enters this latter, to result in the movement of the arm (310) away from said body (300) and release of this latter, this movement taking place away from the longitudinal axis (W) of the pen needle.

4. A pen needle as claimed in claim 3, **characterised in that** the second end (315) of said arm (310) upperly supports a projection of right-angled triangular cross-section with its inclined side (327) facing outwards from the container body (35), said projection (326) cooperating with a closure element (330) for the seat (311) in which said arm (310) is elastically movable, this closure element being disposed in proximity to said end (315) of this latter and being arranged to lock the arm (310) in the position distant from the body (300) by cooperating with the elastic element following its cooperation with said projection (326).

5. A pen needle as claimed in claim 2, **characterised in that** said container body (35) is disposed about a tubular body (33) rigid with the needle carrier (3) and containing the through cannula (5).

6. A pen needle as claimed in claim 1, **characterised in that** the protector element (20) is detached from the container body (35) at the commencement of injection and reaches in proximity to it after the cannula (5) has perforated the patient's epidermis, said protector element (20) moving from a free end (6H) of the cover element (6) towards the needle carrier (3), said movement of the protector element towards said needle carrier (3) continuing after contact with the container body (35), to remove the locking means (300, 310) and compress the elastic element (36) within said container body (35).

7. A pen needle as claimed in claim 1, **characterised by** providing a stable position outside the cover element (6) attained by the protector element (20) when urged by the elastic element (36) on extracting the cannula (5) from the patient, means (21, 80. 101) for stably locking said protector element (20) being provided at the free end (6H) of said cover element (6).

8. A pen needle as claimed in claim 7, **characterised in that** said locking means are a flange (21) present at the second end (20B) of the protector element (20), to cooperate with an edge (22) of the free end (6H) inside the cover element (6).

9. A pen needle as claimed in claim 7, **characterised in that** said locking means are at least one undercut (80) present on an outer surface (81) of the protector element (20) and corresponding with the free end (6H) of the cover element (6).

10. A pen needle as claimed in claim 7, **characterised in that** the locking means are a collar (100) positioned on the tubular body (33) of the pen needle (1) and provided with an undercut (101), said undercut cooperating with a corresponding abutment (102) present in proximity to the second end (20B) of the movable protector element (20).

11. A pen needle as claimed in claim 1, **characterised in that** the protector element (20) comprises on its first end (20A) a plurality of preferably slightly pointed protuberances (30).

## Patentansprüche

1. Stiftnadel, die an einen Behälter oder Stift anzuschließen ist, zu dem eine Ampulle mit Medikament gehört, wobei die Nadel (1) aufweist:
eine Metallkanüle (5), die mit zwei Spitzen (25, 26), einer an jedem Ende, versehen ist, wobei die erste (25), die als "Patientenspitze" bekannt ist, den Zweck hat, die Epidermis zu schneiden, um zu ermöglichen, dass die Kanüle (5) in die Haut des Patienten eingeführt wird, und die zweite (26), die sich innen an einem Nadelträger (3) befindet und als "Ampullenspitze" bekannt ist, den Zweck hat, die medizinische Ampulle zu durchstechen, wenn die Stiftnadel (1) am Stift befestigt wird, wodurch ermöglicht wird, dass das Medikament während der tatsächlichen Injektion von der Ampulle über die Kanüle (5) zu einem Patienten übertragen wird;
den vorgenannten Nadelträger (3), der mit dem Stift zu verbinden ist;
einen primären Behälter (2), der den Zweck hat, die gesamte Stiftnadel (1) vor dem Gebrauch aufzunehmen;
ein Abdeckelement (6), das mit dem Nadelträger (3) verbunden und um die Kanüle (5) herum positioniert ist;
ein Schutzelement (20), das innerhalb des Abdeckelements (6) bewegbar und so angeordnet ist, dass es aus letzterem herausragt, um die erste Spitze der Kanüle (5) nach deren Herausziehen aus dem Patienten bei Beendigung der Injektion abzudecken, um einen versehentlichen Kontakt mit der Spitze (25) durch eine Person, die die Injektion durchführt, oder durch den Patienten selbst zu vermeiden, und um eine Wiederverwendung der Vorrichtung zu verhindern;
ein elastisches Element (36), das angeordnet ist, um auf das Schutzelement (20) zu drücken, um zu ermöglichen, dass es sich bei Beendigung der Injektion und während des Herausziehens der Kanüle (5) aus dem Patienten bewegt, wobei die Bewegung verursacht, dass das Element die erste Spitze (25) der Kanüle (5) abdeckt, wobei Blockiermittel (47, 300, 310) für das elastische Element (36) vorgesehen sind, um seine Aktion auf das Schutzelement (20) zu verhindern, zumindest bis zu einem Zeitpunkt nach dem Beginn des Einführens der Kanüle (5) in die Epidermis, wobei die Blockiermittel (47, 300, 310) nach dem Zeitpunkt, in dem das Einführen begann, entfernbar sind,
**dadurch gekennzeichnet, dass**
das Schutzelement (20) ein erstes Ende (20A) aufweist, das sich außerhalb des Abdeckelements (6) befindet, und ein zweites Ende (20B), das sich innerhalb des letzteren befindet, wobei das Schutzelement (20) wesentlich vom Abdeckelement (6) vorsteht und die Kanüle (5) abdeckt, wenn der Nadelstift einsatzbereit ist (Figur 2, 4), wobei sich das erste Ende (20A) auf der Haut des Patienten befindet, wenn eine Person mit der Injektion beginnt, wobei das Schutzelement (20) innerhalb des Abdeckelements (6) leicht gleitet, um die Kanüle (5) freizulegen, welche somit in die Haut eindringt, bis das zweite Ende (20B) des Schutzes die Blockiermittel kontaktiert, wobei die Blockiermittel (300, 310) entfernt werden, wenn die Nadel in den Körper des Patienten eindringt und das Schutzelement (20) in das Abdeckelement (6) eintritt, wobei die Bewegung dazu führt, dass das elastische Element (36) komprimiert wird, so dass, wenn die Injektion beendet ist, das Entfernen der Nadel (5) aus dem Patienten ermöglicht, dass sich das Schutzelement (20) aufgrund des elastischen Elements (36) relativ zu dem Abdeckelement (6) bewegt, wodurch sich das Schutzelement aus dem Abdeckelement herausbewegt und die Kanüle (5) überlagert.

2. Stiftnadel nach Anspruch 1, **dadurch gekennzeichnet, dass** die Blockiermittel ein innen hohles Behälterelement (35) aufweisen, das wenigstens ein Element (310) aufweist, das in einem entsprechenden Sitz (311), der in einer Wand (312) des Behälterkörpers (35) vorgesehen ist und den inneren Hohlraum des Letzteren definiert, elastisch beweglich ist, wobei das bewegliche Element (310) ein Element zum Blockieren der Bewegung eines Körpers (300) definiert, mit dem ein zweites Ende (36B) des elastischen Elements (36) zusammenwirkt, wobei das bewegliche Blockierelement (310) mit dem Schutzelement (20) zusammenwirkt, wenn es im Abdeckelement (6) positioniert ist, um den Körper (300), der mit dem elastischen Element zusammenwirkt, freizusetzen und zu ermöglichen, dass Letzteres auf den Körper (300) Kraft ausübt, mit einer Folgeaktion des Letzteren auf das Schutzelement, um es an die Außenseite des Abdeckelements (6) zu bewegen und um die erste Spitze oder Patientenspitze (26) der Kanüle (5) herum zu positionieren.

3. Stiftnadel nach Anspruch 2, **dadurch gekennzeichnet, dass** der Körper (300) einen Kragen (301) aufweist, mit dem das zweite Ende (36B) des elastischen Elements (36) zusammenwirkt, wobei sich ein erstes Ende (36A) des elastischen Elements (36) im Behälterkörper (35) befindet und an ihm anliegt, wobei das wenigstens eine bewegliche Element (310) ein Arm ist, der an seinem ersten Ende (313) mit dem Behälterkörper (35) verbunden ist und ein zweites Ende (315) aufweist, das in dem entsprechenden Sitz (311), der in der Wand (312) des Behälterkörpers vorgesehen ist, frei angeordnet ist, wobei das zweite Ende einen Abschnitt (320) aufweist, der winkelig in Richtung des Inneren des Behälterkörpers (35) relativ zu dem entsprechenden Arm (310) gebogen ist und mit Letzterem eine Unterschneidung (322) definiert, die ausgelegt ist, um auf dem Kragen (301) des Körpers (300) aufzuliegen, der mit dem elastischen Element (36) zusammenwirkt, wobei der gebogene Abschnitt (320) eine geneigte Oberfläche (323) aufweist, die sich in Richtung des Inneren des Behälterkörpers (35) verjüngt und mit einem verjüngten Ende (20B) des Schutzelements (20) im Abdeckelement (6) gleitend zusammenwirkt, wobei dieses Zusammenwirken stattfindet, während das Schutzelement (20) wieder in Letzteres eintritt, was zu einer Bewegung des Arms (310) vom Körper (300) weg und zum Freigeben des Letzteren führt, wobei diese Bewegung von der Längsachse (W) der Stiftnadel weg stattfindet.

4. Stiftnadel nach Anspruch 3, **dadurch gekennzeichnet, dass** das zweite Ende (315) des Arms (310) oben einen Vorsprung mit dem Querschnitt eines rechtwinkligen Dreiecks stützt, wobei die geneigte Seite (327) vom Behälterkörper (35) nach außen zeigt, wobei der Vorsprung (326) mit einem Verschlusselement (330) für den Sitz (311), in dem der Arm (310) elastisch bewegbar ist, zusammenwirkt, wobei dieses Verschlusselement nahe dem Ende (315) des Letzteren angeordnet und dazu ausgelegt ist, den Arm (310) in der Position vom Körper (300) entfernt, durch Zusammenwirken mit dem elastischen Element nach seinem Zusammenwirken mit dem Vorsprung (326) festzulegen.

5. Stiftnadel nach Anspruch 2, **dadurch gekennzeichnet, dass** der Behälterkörper (35) um einen rohrförmigen Körper (33), der starr mit dem Nadelträger (3) ist und die durchgehende Kanüle (5) enthält, angeordnet ist.

6. Stiftnadel nach Anspruch 1, **dadurch gekennzeichnet, dass** das Schutzelement (20) zu Beginn einer Injektion vom Behälterkörper (35) abgenommen wird und in seine Nähe reicht, nachdem die Kanüle (5) die Epidermis des Patienten durchstochen hat, wobei sich das Schutzelement (20) von einem freien Ende (6H) des Abdeckelements (6) in Richtung des Nadelträgers (3) bewegt, wobei die Bewegung des Schutzelements in Richtung des Nadelträgers (3) nach dem Kontakt mit dem Behälterkörper (35) andauert, um die Blockiermittel (300, 310) zu entfernen und das elastische Element (36) im Behälterkörper (35) zusammenzudrücken.

7. Stiftnadel nach Anspruch 1, **gekennzeichnet durch** Bereitstellen einer stabilen Position außerhalb des Abdeckelements (6), die **durch** das Schutzelement (20) erzielt wird, wenn von dem elastischen Element (36) beim Herausziehen der Kanüle (5) aus dem Patienten darauf Kraft ausgeübt wird, wobei Mittel (21, 80, 101) zum stabilen Festlegen des Schutzelements (20) am freien Ende (6H) des Abdeckelements (6) vorgesehen sind.

8. Stiftnadel nach Anspruch 7, **dadurch gekennzeichnet, dass** die Blockiermittel ein Flansch (21) sind, der sich am zweiten Ende (20B) des Schutzelements (20) befindet, um mit einer Kante (22) des freien Endes (6H) innerhalb des Abdeckelements (6) zusammenzuwirken.

9. Stiftnadel nach Anspruch 7, **dadurch gekennzeichnet, dass** die Blockiermittel wenigstens eine Unterschneidung (80) sind, die sich auf einer Außenfläche (81) des Schutzelements (20) befindet und mit dem freien Ende (6H) des Abdeckelements (6) zusammenwirkt.

10. Stiftnadel nach Anspruch 7, **dadurch gekennzeichnet, dass** die Blockiermittel ein Kragen (100) sind, der sich auf dem rohrförmigen Körper (33) der Stiftnadel (1) befindet und mit einer Unterschneidung (101) versehen ist, wobei die Unterschneidung mit einem korrespondierenden Anschlag (102) zusammenwirkt, der sich in der Nähe des zweiten Endes (20B) des beweglichen Schutzelements (20) befindet.

11. Stiftnadel nach Anspruch 1, **dadurch gekennzeichnet, dass** das Schutzelement (20) an seinem ersten Ende (20A) eine Vielzahl von bevorzugt leicht zugespitzten Vorsprüngen (30) aufweist.

## Revendications

1. Aiguille-stylo destinée à être reliée à un récipient ou un stylo auquel est associé un flacon de médicament, ladite aiguille (1) comprenant :
- une canule métallique (5) pourvue de deux points (25, 26), un à chaque extrémité : le premier (25), désigné « point patient », ayant pour fonction de couper l'épiderme afin que la canule (5) puisse être introduite dans la peau du patient ; le second (26), situé à l'intérieur d'un porte-aiguille (3) et désigné « point flacon », ayant pour fonction de percer le flacon médicamenteux lorsque l'aiguille-stylo (1) est fixée au stylo, ce qui permet de faire passer le médicament, pendant l'injection proprement dite, du flacon au patient au travers de la canule (5) ;
- le porte-aiguille (3) susmentionné, destiné à être relié audit stylo ;
- un récipient principal (2) dont la fonction est de recevoir l'aiguille-stylo (1) complète avant utilisation ;
- un élément de couverture (6), relié au porte-aiguille (3) et placé autour de la canule (5) ;
- un élément de protection (20), mobile à l'intérieur de l'élément de couverture (6) et conçu pour ressortir de ce dernier de manière à couvrir le premier point de la canule (5) après son extraction du patient une fois l'injection terminée, afin d'éviter tout contact accidentel avec ledit point (25) par un opérateur effectuant l'injection ou par le patient lui-même, et afin de prévenir toute réutilisation du dispositif ;
- un élément élastique (36) conçu pour appuyer sur ledit élément de protection (20) de manière à lui permettre de se déplacer à la fin de l'injection et pendant l'extraction de la canule (5) du patient, ledit déplacement amenant ledit élément à couvrir le premier point (25) de ladite canule (5), des moyens de blocage (47, 300, 310) étant prévus pour ledit élément élastique (36) de manière à prévenir son action sur ledit élément de protection (20), au moins jusqu'à un moment consécutif au commencement de l'introduction de la canule (5) dans l'épiderme, lesdits moyens de blocage (47 ; 300, 310) pouvant être retirés après ledit moment auquel ladite introduction a commencé, **caractérisé en ce que** l'élément de protection (20) comprend une première extrémité (20A) située à l'extérieur dudit élément de couverture (6) et une seconde extrémité (20B) située à l'intérieur de ce dernier, l'élément de protection (20A) ressortant très nettement de l'élément de couverture (6) et couvrant la canule (5) lorsque le stylo-aiguille est prêt à l'usage (figure 2, 4), ladite première extrémité (20A) se trouvant sur la peau du patient lorsqu'un opérateur commence l'injection, ledit élément de protection (20) coulissant facilement à l'intérieur de l'élément de couverture (6) pour exposer la canule (5) qui pénètre de ce fait dans la peau jusqu'à ce que la seconde extrémité de protection (20B) vienne au contact des moyens de blocage, lesdits moyens de blocage (300, 310) étant retirés lorsque l'aiguille pénètre dans le corps du patient et l'élément de protection (20) entre dans l'élément de couverture (6), ledit déplacement comprimant l'élément élastique (36) de sorte que, lorsque l'injection est terminée, le retrait de l'aiguille (5) du patient permet à l'élément de protection (20) de se déplacer par rapport audit élément de couverture (6), l'élément élastique (36) le laissant faire, ledit élément de protection sortant alors de l'élément de couverture et recouvrant la canule (5).

2. Aiguille-stylo selon la revendication 1, **caractérisée en ce que** les moyens de blocage comprennent un élément formant récipient creux (35) présentant au moins un élément (310) pouvant se déplacer de manière élastique à l'intérieur d'un siège correspondant (311) prévu dans une paroi (312) dudit corps de récipient (35) qui délimite la cavité interne de ce dernier, ledit élément mobile (310) définissant un élément destiné à bloquer le déplacement d'un corps (300) avec lequel coopère une seconde extrémité (36B) dudit élément élastique (36), ledit élément de blocage mobile (310) coopérant avec l'élément de protection (20) lorsqu'il se trouve à l'intérieur de l'élément de couverture (6), de manière à libérer le corps (300) coopérant avec l'élément élastique et à permettre à ce dernier de pousser ledit corps (300), avec une action consécutive de ce dernier sur ledit élément de protection de manière à le déplacer vers l'extérieur de l'élément de couverture (6) et à le placer autour du premier point ou point patient (25) de la canule (5).

3. Aiguille-stylo selon la revendication 2, **caractérisée en ce que** ledit corps (300) présente un collier (301) avec lequel coopère la seconde extrémité (36B) de l'élément élastique (36), une première extrémité (36A) dudit élément élastique (36) se trouvant à l'intérieur du corps de récipient (35) et reposant sur celui-ci, ledit au moins un élément mobile (310) étant un bras relié à sa première extrémité (313) audit corps de récipient (35) et ayant une seconde extrémité (315) placée librement à l'intérieur du siège correspondant (311) prévu dans la paroi (312) dudit corps de récipient, ladite seconde extrémité présentant une partie (320) fléchie vers l'intérieur dudit corps de récipient (35) par rapport au bras correspondant (310) et définissant avec ce dernier une contre-dépouille (322) conçue pour reposer sur le collier (301) du corps (300) coopérant avec ledit élément élastique (36), ladite partie fléchie (320) présentant une surface inclinée (323) s'effilant vers l'intérieur du corps de récipient (35) et coopérant de façon coulissante avec une extrémité effilée (20B) de l'élément de protection (20) situé à l'intérieur de l'élément de couverture (6), cette coopération ayant lieu lorsque l'élément de protection (20) rentre dans ce dernier, pour provoquer le déplacement (310) à l'écart dudit corps (300) et la libération de ce dernier, ce déplacement ayant lieu à l'écart de l'axe longitudinal (W) de l'aiguille-stylo.

4. Aiguille-stylo selon la revendication 3, **caractérisée en ce que** la seconde extrémité (315) dudit bras (310) porte dans sa partie supérieure une saillie de section triangulaire à angle droit dont le côté incliné (327) est orienté vers l'extérieur du corps de récipient (35), ladite saillie (326) coopérant avec un élément de fermeture (330) du siège (311) dans lequel ledit bras (310) peut se déplacer de manière élastique, cet élément de fermeture se trouvant à proximité de ladite extrémité (315) de ce dernier et étant conçu pour bloquer le bras (310) dans la position distante du corps (300) en coopérant avec l'élément élastique après sa coopération avec ladite saillie (326).

5. Aiguille-stylo selon la revendication 2, **caractérisée en ce que** ledit corps de récipient (35) est placé autour d'un corps tubulaire (33) solidaire du porte-aiguille (3) et contenant la canule de traversée (5).

6. Aiguille-stylo selon la revendication 1, **caractérisée en ce que** l'élément de protection (20) se détache du corps de récipient (35) au commencement de l'injection et arrive à proximité de ce dernier après que la canule (5) a percé l'épiderme du patient, ledit élément de protection (20) se déplaçant à partir d'une extrémité libre (6H) de l'élément de couverture (6) vers le porte-aiguille (3), ledit déplacement de l'élément de protection vers ledit porte-aiguille (3) se poursuivant après mise en contact avec le corps de récipient (35), pour retirer les moyens de blocage (300, 310) et comprimer l'élément élastique (36) à l'intérieur dudit corps de récipient (35).

7. Aiguille-stylo selon la revendication 1, **caractérisée en ce qu'**elle permet à l'élément de protection (20) de parvenir à une position stable à l'extérieur de l'élément de couverture (6) lorsqu'il est poussé par l'élément élastique (36) lors de l'extraction de la canule (5) du patient, des moyens (21, 80, 101) destinés à bloquer de façon stable ledit élément de protection (20) étant prévus à l'extrémité libre (6H) dudit élément de couverture (6).

8. Aiguille-stylo selon la revendication 7, **caractérisée en ce que** lesdits moyens de blocage sont une bride (21) située sur la seconde extrémité (20B) de l'élément de protection (20), pour coopérer avec un bord (22) de l'extrémité libre (6H) à l'intérieur de l'élément de couverture (6).

9. Aiguille-stylo selon la revendication 7, **caractérisée en ce que** lesdits moyens de blocage sont au moins une contre-dépouille (80) située sur une surface extérieure (81) de l'élément de protection (20) et correspondant à l'extrémité libre (6H) de l'élément de couverture (6).

10. Aiguille-stylo selon la revendication 7, **caractérisée en ce que** les moyens de blocage sont un collier (100) placé sur le corps tubulaire (33) de l'aiguille-stylo (1) et pourvu d'une contre-dépouille (101), ladite contre-dépouille coopérant avec une butée correspondante (102) située à proximité de la seconde extrémité (20B) de l'élément de protection mobile (20).

11. Aiguille-stylo selon la revendication 1, **caractérisée en ce que** l'élément de protection (20) comprend, sur sa première extrémité (20A), une pluralité de protubérances de préférence légèrement pointues (30).
